# EUROPEAN PATENT SPECIFICATION

(11) **EP 2 622 075 B1**
(45) Date of publication and mention of the grant of the patent: **02.03.2016**
(21) Application number: 10777121.4
(22) Date of filing: 27.09.2010
(51) Int. Cl.: C12N 15/113, C12Q 1/68, A61K 31/7105

(54) **USE OF MIRNAS FOR THE DIAGNOSIS, PROPHYLAXIS, TREATMENT AND FOLLOW-UP OF DISEASES INVOLVING MACROAUTOPHAGY ABNORMALITIES**
VERWENDUNG VON MIRNA ZUR DIAGNOSE, PROPHYLAXE, BEHANDLUNG UND NACHSORGE VON ERKRANKUNGEN MIT MAKROAUTOPHAGISCHEN ANOMALIEN
UTILISATION D'ARNMI POUR DIAGNOSTIQUER, PRÉVENIR, TRAITER ET SUIVRE LES MALADIES IMPLIQUANT DES ANOMALIES DE TYPE MACRO-AUTOPHAGIE

(43) Date of publication of application: 07.08.2013
(73) Proprietor: Sabanci Universitesi, 34956 Istanbul (TR)
(72) Inventor: GOZUACIK, Devrim, 34956 Istanbul (TR); KORKMAZ, Gozde, 34956 Istanbul (TR)
(74) Representative: Sevinç, Erkan
(86) International application number: PCT/TR2010/000192
(87) International publication number: WO 2012/044265

(56) References cited:
- WO-A1-2007/051316
- WO-A2-2007/103808
- WO-A2-2010/055487
- US-A1- 2010 010 072
- ZHU HUA ET AL: "Regulation of autophagy by a beclin 1-targeted microRNA, miR-30a, in cancer cells.", AUTOPHAGY AUG 2009 LNKD- PUBMED:19535919, vol. 5, no. 6, August 2009 (2009-08), pages 816-823, XP002637509, ISSN: 1554-8635 cited in the application
- KOUKOURAKIS M I ET AL: "Beclin 1 over- and underexpression in colorectal cancer: distinct patterns relate to prognosis and tumour hypoxia.", BRITISH JOURNAL OF CANCER 12 OCT 2010 LNKD- PUBMED:20842118, vol. 103, no. 8, 14 September 2010 (2010-09-14), pages 1209-1214, XP002637510, ISSN: 1532-1827

## Description

### Technical Field

The invention is especially related to use of hsa-miR-376b and inhibitors of said miRNA for diagnosis, prophylaxis, treatment, and follow-up during and after treatment of at least one disease involving autophagy abnormalities by controlling autophagy-related gene expression and methods thereof.

### The Related Art

Autophagy is characterized by sequestration of bulk cytoplasm, proteins and organelles in double or multimembrane vesicles, and their delivery to and subsequent degradation by the cell's own lysosomal/vacuolar system (Kim *et al.,* 2002). This biological phenomenon occurs at low basal levels in all cell types (from yeast to mammals) under non-deprived conditions, performing homeostatic functions like protein degradation and organelle turnover. Autophagy is rapidly upregulated under conditions leading to cellular stress like nutrient or growth factor deprivation, to provide an alternative source of intracellular building blocks and substrates for energy generation (Kim *et al.,* 2002).

Although initial morphology-based studies were performed in mammalian cells and tissues, genes regulating autophagy were discovered in the yeast. At least 30 ATG (autophagy) genes (previously called APG, AUT, and/or CVT genes) were cloned from the yeast and their protein products were shown to play a role in various stages of the autophagic process, including vesicle enucleation, expansion, autophagic vesicle fusion to late endosome/lysosome, and cargo degradation (Gozuacik and Kimchi 2004, Ohsumi 2001). Mammalian orthologues of some of these genes have been cloned, and their function was indeed preserved in the mammalian system (Mizushima *et al.,* 2002).

Similar to the yeast model, mammalian autophagy pathways proceed through the action of various protein complexes. For example, Atg1/Ulk1-2 protein complexes relay signals from the growth factor signals and nutritional status sensor Torc1 complex to the autophagy pathway (Vellai T *et al.,* 2009). Inactivation of the key protein in the complex, mTor, leads to the stimulation of autophagy. Another important event in autophagic vesicle formation is the production and accumulation of phosphatidyl-inositol 3-phosphate by a Vps34 phosphoinositol 3-kinase complex in the autophagic vesicle nucleation centers called isolation membranes (Furuya N. *et al.,* 2005). Vps34 complex activity is turned on by several proteins including Beclin1. Being one of the key proteins in autophagy pathways, Beclin1 mRNA and protein levels are generally upregulated during autophagy activation (Rami *et al.,* 2008) and, knockout or knockdown of this protein inhibit autophagy in various systems.

Two ubiquitin-like conjugation systems are instrumental in autophagic vesicle biogenesis. The first consists of the Atg12 system in which Atg12 is conjugated to Atg5 in a ubiquitination-like fashion (Mizushima *et al.,* 1998). The conjugation reaction starts by the activation of Atg12 by an E1-like enzyme called Atg7. This is followed by the transfer of Atg12 by Atg7 to an E2-like enzyme called Atg10 (Shintani *et al.,* 1999; Tanida *et al.,* 1999). Finally, Atg12 is conjugated to Atg5 by the covalent linkage of a carboxyterminal glycine of Atg12 to a lysine residue in the central part of the Atg5 protein. The Atg12/Atg5 conjugation is functional in the formation and stabilization of a larger protein complex, containing Atg12/Atg5 and Atg16. (Mizushima *et al.,* 1999; Mizushima *et al.,* 2003a). Atg16 (Atg16L in mammalians) has the capacity to homo-oligomerize through its coiled coil domain. The Atg12/Atg5/Atg16 complex serves as an E3-like enzyme for the second ubiquitination-like reaction.

In the second reaction Atg8 (or mammalian LC3) is conjugated to a lipid molecule, phosphatidylethanolamine (PE) (Ichimura *et al.,* 2000). For this conjugation reaction to occur, the Atg8/LC3 protein has to be processed by the cleavage of a portion of its carboxy-terminus at the time of its translation. The proteases responsible for this processing reaction are the Atg4 proteins (Atg4a-c) (Kirisako *et al.,* 2000). Cleavage of Atg8/LC3 by Atg4 exposes a carboxy-terminal glycine. Following the action of the E1-like protein Atg7, and the E2-like protein Atg3, the carboxy-terminal glycine of Atg8/LC3 protein is conjugated to an amino group of the PE. This conjugation causes the otherwise cytoplasmic/peripherally membrane-bound form of Atg8/LC3, to tightly associate with membranes (Kirisako *et al.,* 1999). The lipidation reaction is reversible and Atg4 proteases, also acting like ubiquitin deconjugating enzymes, are capable of cleaving the amide bond between Atg8/LC3 and PE, enabling the recycling of Atg8/LC3.

Atg9 protein recycling and interaction with proteins such as Atg18 and Atg2 is also considered as important events in autophagic vesicle nucleation and elongation through their possible role in the transport of lipid molecules (Reggiori F *et al.,* 2004).

Through a coordinated action of above mentioned protein complexes, autophagic vesicles are nucleated, their membranes are elongated, the cargos are recruited and the double membrane autophagosomes are formed. Autophagosomes fuse with late endosomes or lysosomes to form autolysosomes. Specific factors have been implicated in this step. Vps complex and Rab GTPases proteins are involved in the organization of the fusion site. Then, SNAREs proteins (SNAP as soluble NSF attachment protein receptor) (Darsow T. 1997) form a complex which serves as a bridge between the two organelles. Membranes fuse to form autolysosomes and the cargo is delivered to the lysosomal machinery.

In the lumen of lysosome/vacuole, lipases such as Atg15 first degrade the remaining autophagic membrane and the cargo is then catabolized by lysosomal lytic enzymes (Kim *et al.,* 2007). Following the degradation of the vesicle, building blocks are carried to the cytosol for further use. Specialized lysosome membrane proteins play a role in this process including lysosomal membrane proteins LAMP-1 and LAMP-2.

Autophagy also plays a critical role in several diseases including liver and muscle disorders, neurodegenerative diseases, hearth diseases, ageing, myopathies, auto-immune and inflammatory diseases infectious diseases, ischemic diseases, immune deficiencies, diabetes, axonal injury, lysosomal storage diseases, nervous system diseases, kidney diseases and cancer (Mizushima *et al.,* 2008, Cuervo A.M. 2004). In the case of neurodegenerative diseases, there are indications that autophagy may play a role in elimination of accumulated abnormal proteins to restore homeostasis and/or it may contribute to neuronal cell death (Gozuacik and Kimchi 2004, Shintani and Klionsky 2004). In cancer, autophagy and probably autophagic cell death are important tumor suppressor mechanisms that are inactivated during tumor formation (Gozuacik and Kimchi 2004, Ohsumi 2001). Paradoxically, depending on the tumor type, autophagy may also play a role in tumor cell survival under ischemic conditions imposed by rapid growth and defective vascularization. Furthermore, autophagy constitutes an important cellular defense system against intracellular parasitic organisms including bacteria and viruses (Shintani and Klionsky 2004). Therefore, research on the mechanisms of autophagy and its relation to pathological conditions is of outmost importance.

Recent studies directly implicated some autophagy-related proteins in diseases such as cancer and Crohn's disease. For instance, Beclin 1 is a well-known example of cancer related autophagy genes. Heterozygous deletion of Beclin1 in mice led to an increase in both spontaneous and induced tumor formation rate. Monoallelic Beclin1 deletion was observed in 40-75% of breast and ovarian cancers (Aita *et. al.,* 1999). In human breast epithelial carcinoma cells, endogenous Beclin 1 protein expression was found at low levels. Thus, decreased expression of this protein seemed to contribute to the development/progression of breast malignancies (Liang *et*. *al.,* 1999). Decreased Beclin1 expression was also observed in cervical aquamous carcinoma and epithelial ovarian carcinoma cells (Wang *et. al.,* 2006; Wang *et. al.,* 2007). A study based on Beclin1 protein and mRNA expression levels in human brain tumors showed that Beclin1 was downregulated in brain tumors (Miracco *et. al.,* 2007). Another study revealed that Beclin1 gene inhibits tumor growth in colon cancer cell lines depending on the mRNA and protein expression levels (Koneri *et. al.,* 2007). Abnormalities in the genes of autophagy proteins Atg16L and IRGM, were found to be associated with Crohn's disease (an inflammatory bowel disease) (Palomino-Morales RJ *et al.,* 2009). Further studies suggested that Atg16L or Atg5 deficient bowels harboured abnormal Paneth cells contributing to mucosal defense abnormalities observed in Crohn's diseased individuals (Cadwell K *et al*., 2008).

miRNAs are one of the negative regulators of the gene expression controlling various fundamental biological processes such as cell proliferation, stem cell division, and apoptosis (Ambros, V., 2004). miRNAs act either by altering the stability of the mRNA transcripts and/or blocking protein translation by ribosomes (Lewis, B.P., et al, 2005). miRNAs are synthesized as pri-miRNAs from miRNA gene clusters. The sizes of pri-miRNAs vary between a few hundred to a few thousand nucleotides. In the nucleus, processing of pri-miRNA by Drosha and Pasha/DGCR8 results in the production of around 60-70 bases long pre-miRNA, composed of a stem and loop structure with flanking sequences. Following transport into the cytoplasm by exporting 5 and Ran-GTP, processing of pre-miRNA by Dicer proteins results in the production of small RNA duplexes of around 22 nucleotides. The guiding strand of the miRNA duplex (ds-miRNA) is then loaded into the RNA-induced silencing complex (RISC). RISC guided by the mature miRNA will then target the mRNAs through miRNA-binding sites frequently found in their 3'UTR regions, leading to their translational repression or degradation. By this way, miRNAs regulate transcription and/or translation of their target genes and proteins and orchestrate physiological and pathological events.

Up to now, there is only one published study describing a relationship between miRNAs and autophagy in the mammalian system. In this study, Zhu et al demonstrated a direct interaction between Beclin 1 gene and miR-30a (Zhu *et al* 2009).

A better understanding of miRNA-autophagy relationship will contribute to the control diseases involving autophagy abnormalities. Some of the reasons for using miRNAs as potential therapeutic targets are as follows;
- miRNAs are organic molecules and natural antisense interactors;
- miRNA expression profiles can be used to diagnose a disease state and the deregulated miRNAs contribute to the initiation and development of diseases;
- miRNAs have the capacity to regulate and modulate biological and pathological processes by orchestrating the expression of several genes at once;
- mouse models mimicking human disease showed that miRNAs play an important role in different diseases and might be used as therapeutic molecules (Melnikova I 2007);
- their small size (22-24 nucleotides in length) and their organic nature make them very attractive for drug development.

If the related art is searched in patent literature, there can be obtained several applications related to miRNAs and diseases. For instance, American patent application with the number of US 20100113577 discloses that miR145 nucleic acids specifically bind the 3' UTR within endogenous IRS-I such as to suppress or inhibit colon cell proliferation. Therefore it can be used in treating the colon cancer. Additionally, patent application numbered US 20100010073 writes about usage of microRNAs (miRNAs) for the diagnosis, prophylaxis and/or treatment of heart diseases. Moreover, it is mentioned in American patent application, US 20100048674, that the ability of miR-181a to support active signaling between Notch and pre-TCR pathways by coordinately dampening negative regulators of these pathways allows the use of miR-181a as a therapeutic target for T-lineage acute lymphoblastic leukemia. However, all these applications do not disclose any disease treating relation between miRNA and autophagy.

The current application presents findings that are the first to demonstrate the regulation of the autophagic pathway at the multiple levels *via* specific miRNA family or inhibitors thereof.

The declared specific miRNA family is hsa-miR-376 family. miR-376 family is coded by a miRNA cluster region in the human chromosome 14q32, named Dlkl/Gtl2 region. miR-376 homologues exist in mice as well and they are located to the distal end of the mouse chromosome 12 (Kircher M *et al.,* 2008, and Seitz H *et al.,* 2004). miR-376 RNAs are expressed in various embryonic and adult tissues (Seitz H *et al.,* 2004, and Poy M N *et al.,* 2004).

WO 2010/055487 A2 discloses compositions and methods for microRNA (miRNA) expression profiling of colorectal cancer. It further discloses a diagnostic kit of molecular markers for identifying one or more mammalian target cells exhibiting or having a predisposition to develop colorectal cancer. The kit comprises a plurality of nucleic acid molecules, each nucleic acid molecule encoding a miRNA sequence, wherein one or more of the plurality of nucleic acid molecules are differentially expressed in the target cells and in one or more control cells.

US 2010/010072 A1 discloses molecular markers for diagnosis and classification of human breast cancer and lung cancer. It provides miRNA sequences which are useful as forming the basis for molecular targeting for cancer intervention, in which for example LNA-modified, stabilized synthetic miRNA species (miRNA agonist) are used to replace the activity of a missing or a downregulated miRNA or an LNA-modified antagonistic-miR oligonucleotide used to inhibit the activity of an amplified or over-expressed miRNA.

WO 2007/103808 A2 discloses methods for diagnosing whether a subject has, or is at risk of developing, pancreatic cancer. The methods include measuring the level of at least one miR gene product in a biological sample derived from the subject's pancreas. An alteration in the level of the miR gene product in the biological sample as compared to the level of a corresponding miR gene product in a control sample, is indicative of the subject either having, or being at risk for developing, pancreatic cancer.

The main aim of the invention is to block autophagy in cells. Thus, diseases arising due to excess autophagy can be diagnosed, prevented or treated. On the other hand, another aspect of the invention is initiation of autophagy by suppressing miRNAs being subject of the invention with inhibitors of said miRNAs to control the diseases occurring because of deficiencies on autophagy of the cell.

The invention is especially related to the use of miRNAs and inhibitors of said miRNAs for diagnosis, prophylaxis, treatment, and follow-up during and after treatment of at least one disease involving autophagy abnormalities by controlling autophagy-related gene expression and methods thereof.

### Short description of figures

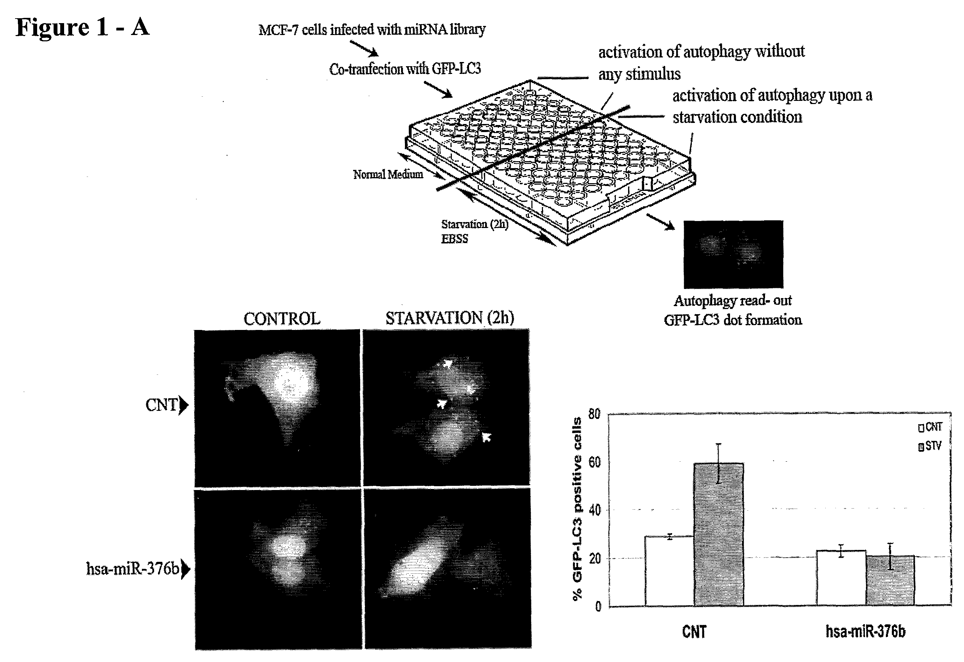

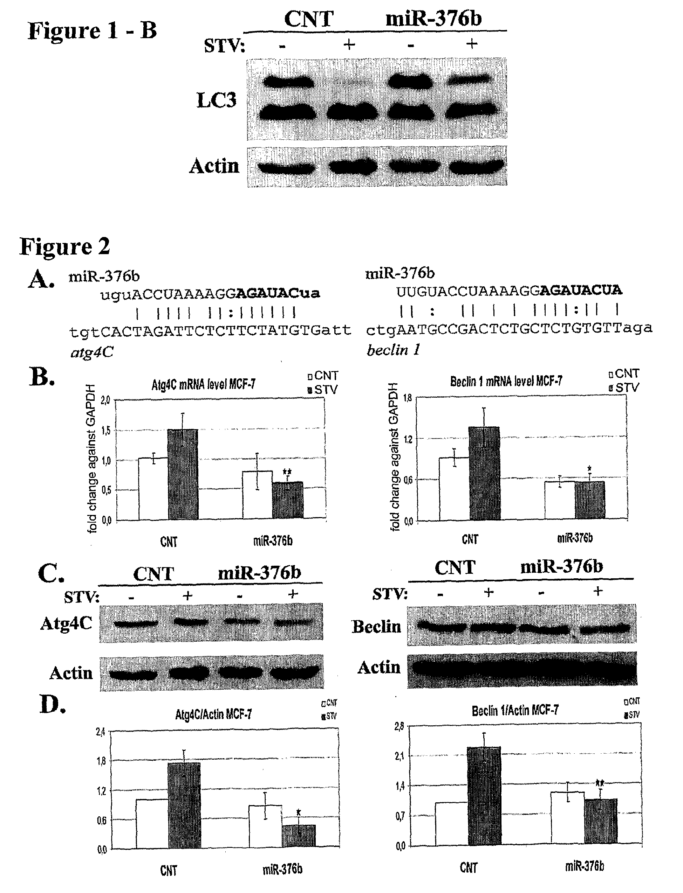

**Figure 1****.** hsa-miR-376b overexpression is able to block autophagy.
   **A.** miRNA screen set-up. Upper panel: MCF-7 cells were transiently transfected with miRNAs individually in a 96-well plate system. Each miRNA was tested in duplicate. Starvation was induced by replacing full medium with EBSS. Lower panel: Percentage of GFP-LC3 puncta formation after 2 hours of starvation was observed. For each condition, 150-200 cells were counted. Graphical representation of percentage of GFP-LC3 puncta positive cells indicated a 2-fold increase from the control level following starvation (STV); but this increase was inhibited by miRNA over-expression (n=4).
   **B.** Autophagy-associated LC3-II formation was attenuated by miR-376b. Decrease in free LC-I form and accumulation of LC3-II form indicated autophagy activation following starvation (STV) in control miRNA transfected cells (CNT). In contrast, miR-376b overexpression resulted in the reversal of the process and led to the inhibition of autophagy, reflected by the presence of a higher level of LC3-I and relatively lower level of LC-II both under non-starved, and in a more pronounced way, under starved (STV) conditions.
**Figure 2****.** miR-376b target Atg4c and Beclin 1 in MCF-7 breast carcinoma cells.
   **A.** Scheme representing putative miR-376b binding sequences (miR Response Element, MRE) in the 3' UTR of Atg4c and Beclin1 (Identified by SANGER algorithm.)
   **B.** Quantitative PCR (qPCR) analysis of Atg4c and Beclin1 mRNA levels in control (CNT) or miR-376b transfected (miR-376b) MCF-7 cells. mRNA level of both Atg4c (left) and Beclin1 (right) was significantly decreased following miR-376b expression under both non-starved and starved conditions (n=3). qPCR data was normalized using GAPDH mRNA levels.
   **C and D.** Atg4c and Beclin1 protein levels were attenuated following miR-376b expression. **C.** Immunoblots of control (CNT) or miR-376b transfected cells (miR-376b) that were non-starved or starved (STV). β-Actin was used as loading control.
   **D.** Densitometric analysis of immunoblots in C using ImageJ software. (n=5 for Beclin 1, n=3 for Atg4C)

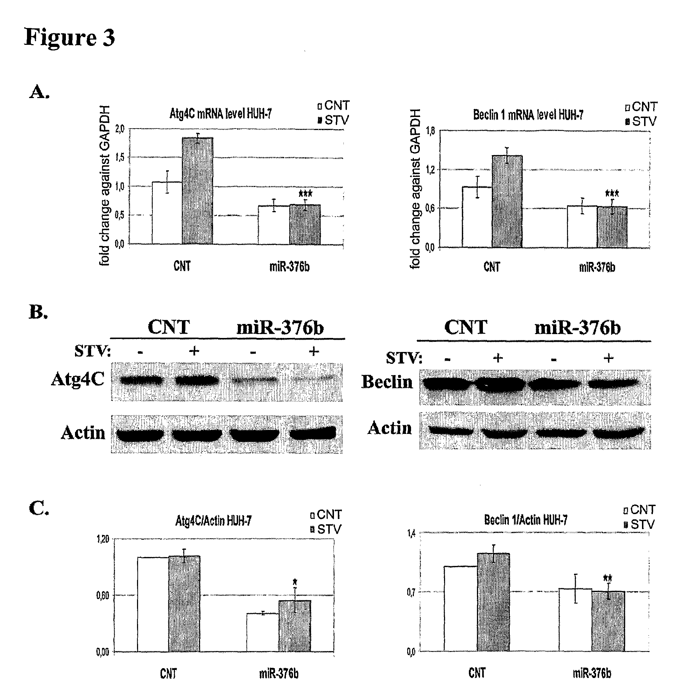

**Figure 3****.** miR-376b target Atg4c and Beclin 1 in Huh-7 hepatocarcinoma cells.
   **A.** Quantitative PCR (qPCR) analysis of Atg4c and Beclin1 mRNA levels in control (CNT) or miR-376b transfected (miR-376b) Huh-7 cells. mRNA level of both Atg4c (left) and Beclin1 (right) was significantly decreased following miR-376b expression under both non-starved and starved conditions (n=3). qPCR data was normalized using GAPDH mRNA levels.
   **B and C.** Atg4c and Beclin1 protein levels were attenuated following miR-376b expression. **B.** Immunoblots of control (CNT) or miR-376b transfected cells (miR-376b) that were non-starved or starved (STV). β-Actin was used as loading control.
   **C.** Densitometric analysis of immunoblots in C using ImageJ software. (n=3 for both Beclin 1 and Atg4C)

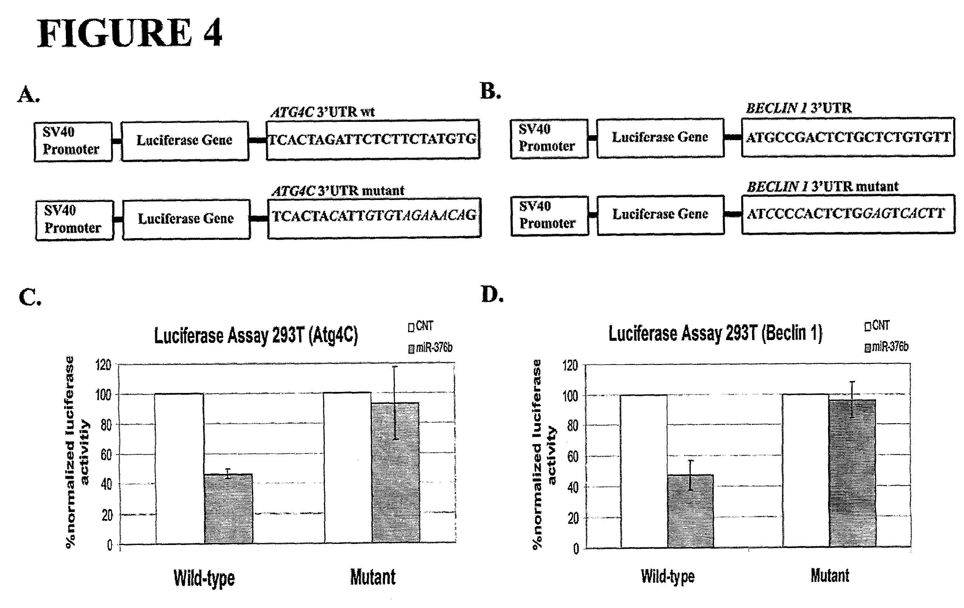

**Figure 4****.** Putative miR-376-binding sites are involved in the observed miRNA effect on Atg4c and Beclin1.
   **A.** Scheme representing sequences of Atg4c MRE (top sequence) or its mutated form (bottom sequence) cloned in the 3' UTR of the reporter firefly luciferase in the pGL3 vector.
   **B.** Scheme representing sequences of Beclin1 MRE (top sequence) or its mutated form (bottom sequence) cloned in the 3' UTR of the reporter firefly luciferase in the pGL3 vector.
   **C.** Normalized luciferase activity in extracts from cells co-transfected with the wild-type or mutant Atg4c luciferase constructs and control (CNT) or miR-376 (miR-376) plasmids.
   **D.** Normalized luciferase activity in extracts from cells co-transfected with the wild-type or mutant Beclin1 luciferase constructs and control (CNT) or miR-376 (miR-376) plasmids. Luciferase activities were normalized to the co-transfected renilla luciferase activity.
**Figure 5****.** Anti miRNAs (antagomirs) against endogenous miR-376b led to an increase in Atg4c and Beclin1 mRNA levels. Graph representing Atg4c (left) and Beclin1 (right) mRNA levels in non-starved (No-STV) cells tranfected with control (CNT) antagomirs or antagomirs against miR-376b (antagomir). Results were expressed as fold change against GAPDH mRNA levels.

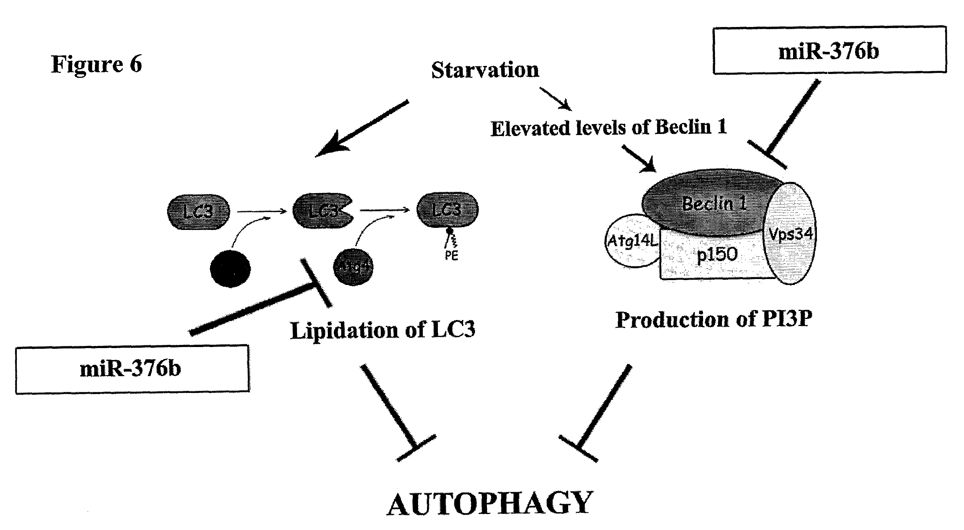

**Figure 6****.** Schematic representation of miR-376b action in autophagy.

### Detailed Description of the Invention

The invention involves the use of at least one miRNA (micro Ribonucleic Acid) being member of the miR-376 family or at least one inhibitor of said miRNA for the diagnosis, prophylaxis, treatment and follow-up during and after treatment of at least one disease involving autophagy abnormalities alone or in combination with other conventional treatments of diseases.

The invention is also related to autophagy control method to provide diagnosis, prophylaxis, treatment and follow-up during and after treatment of at least one disease involving autophagy abnormalities. The steps of said method are;
• providing at least one miRNA being member of the miR-376 family or at least one inhibitor for miRNA being member of said family into the cell in which said autophagy occurs, and
• inhibition or initiation of autophagy by the help of said miRNA or inhibitor thereof, where said miRNA inhibitor is a nucleic acid molecule comprising a sequence that is at least 70% complementary to a contiguous 5' to 3' sequence of a mature miRNA,.

In the method, when the disease is originated from excessive autophagy, it comprises the steps of;
• providing excess amount of at least one miRNA being member of the miR-376 family into the cell or organism in which said excess autophagy occurs,
• suppressing at least one autophagy related gene,
• inhibition of excess autophagy by the help of keeping said gene under control.

If the disease is originated from the absence or deficiency of autophagy, then the method comprises the steps of;
• providing excess amount of at least one inhibitor for miRNA being member of the miR-376 family into the cell which has deficiency of autophagy, where said miRNA inhibitor is a nucleic acid molecule comprising a sequence that is at least 70% complementary to a contiguous 5' to 3' sequence of a mature miRNA,
• suppressing said miRNA,
• preventing autophagy related gene(s) from being suppressed,
• initiation of autophagy by the help of said autophagy related gene.

Hereinabove, the miRNA is from 12 nucleotides to 170 nucleotides in length. Said miRNA is a naked synthetic RNA or a chemically modified synthetic RNA. If it is latter, it is modified with a chemical moiety selected from the group consisting of phosphorothioate, boranophosphate, 2'-O-methyl, 2'-fluoro, , 2'-O-methoxyethyl (2'-O-MOE), 2'-O-aminopropyl (2'-O-AP), 2'-O-dimethylaminoethyl (2'-O-DMAOE), 2'-O-dimethylaminopropyl (2'-O-DMAP), 2'-O-dimethylaminoethyloxyethyl (2'-O-DMAEOE), and 2'-O-N-methylacetamido (2'-O-NMA), PEG, terminal inverted-dT base, fluoro-β-d-arabinonucleic acid (FANA or as 4'-S-FANA) or arabinonucleic acid (ANA) modifications, the addition of lauric acid, lithocholic acids and cholesterol derivatives and combinations thereof.

The mentioned miRNA of miR-376 family comprises pri-miRNA, pre-miRNA, mature miRNAs, miRNA seed sequence, dsmiRNA and fragments or variants thereof in a sequence of nucleotide as set forth in the SEQ ID NO:1 and No:2. Said miRNA is preferably miR-376b or RNA complementary of any of the sequences in RNA with a sequence at least about %70 identical to 21 contiguous nucleotides of the miRNA (e.g. SEQ ID NO:2).

On the other hand, said miRNA inhibitors are at least one nucleic acid molecules (for instance, antagomirs) that target the miRNAs of miR-376 family. miRNA inhibitor molecule that is between 12 and 30 nucleotides in length and comprises a 5' to 3' sequence that is at least 70% complementary to a contiguous 5' to 3' sequence of a pri-miRNA, pre-miRNA, mature miRNAs, miRNA seed sequence, dsmiRNA and fragments or variants.

Said miRNA inhibitor comprises a modification selected from the group consisting of 2'-deoxy, 2'-deoxy-2'-fluoro, 2'-O-methyl, 2'-O-methoxyethyl (2'-O-MOE), 2'-O-aminopropyl (2'-O-AP), 2'-O-dimethylaminoethyl (2'-O-DMAOE), 2'-O-dimethylaminopropyl (2'-O-DMAP), 2'-O-dimethylaminoethyloxyethyl (2'-O-DMAEOE), and 2'-O-N-methylacetamido (2'-O-NMA), PEG, terminal inverted-dT base, fluoro-β-d-arabinonucleic acid (FANA or as 4'-S-FANA) or arabinonucleic acid (ANA) modifications, the addition of lauric acid, lithocholic acids and cholesterol derivatives and combinations.

Both miRNA and inhibitors thereof are preferably encoded by an isolated nucleic acid. DNA molecules encoding the miRNA, miRNA precursor molecules or anti miRNAs are also within the scope of the invention. The nucleic acids may be selected from RNA, DNA or nucleic acid analog molecules, such as sugar- or backbone-modified ribonucleotides or deoxyribonucleotides. It should be noted, however, that other nucleic analogs, such as peptide nucleic acids (PNA) or locked nucleic acids (LNA), are also suitable. The isolated nucleic acids may be integrated into a vector and the vector is selected from the group consisting of a plasmid, cosmid, phagemid, virus and artifical chromosome.

The autophagy-related gene which is suppressed by the miRNAs is at least one of Beclin 1 (Atg6) and Atg4C. The disease desired to be diagnosed, prevented or treated is selected from a group consisting of neurodegeneration, cancer, hearth diseases, liver diseases, ageing, myopathies, auto-immune, inflammatory diseases, infectious diseases, ischemic diseases, immune deficiencies, diabetes, axonal injury, lysosomal storage diseases, nervous system diseases. The presented experimental procedures and obtained results below prove the effects especially on cancer cells, especially breast and liver.

When said miRNAs are intended to be used on diseased tissues, they can be administered in a liposome, polymer-based nanoparticle, cholesterol conjugate, cyclodextran complex, polythylenimine polymer, a protein complex or integrated to a virus or virus-like particle, intravenously, subcutaneously, intramuscularly, nasally, intraperitonealy, vaginally, anally, orally, intraocularly or intrathecally.

### Experimental procedure

To discover miRNAs regulating autophagy, an unbiased screen in MCF-7 breast cancer cells was performed by using a GFP fused Atg8/LC3 (GFP-LC3) as an autophagy marker. MCF-7 breast cancer cells were cultured in Dulbecco's modified Eagle's medium (DMEM, Biological Industries) supplemented with 10% fetal calf serum (FCS) and antibiotics (Peniciline/Streptomycin) at 37°C and 5% CO2.

Then cells were co-transfected with the GFP-LC3 marker plasmid together with a plasmid carrying one of the 443 human miRNAs or the control hTR (human telomerase mRNA). In cells grown in full medium, GFP fused LC3 showed a diffuse cytoplasmic/nuclear staining. Following an autophagy stimulus such as serum, amino acid and glucose starvation provided here by the usage of a balanced buffer solution without any additives (EBSS), GFP-LC3 puncta appeared in the cytoplasm reflecting the recruitment of LC3 proteins to autophagic vesicles. Human miRNAs were transfected to cells in duplicates and miRNAs consistently blocking starvation-induced GFP-LC3 dot formation (Fig. 1-A), hence autophagy, were selected.

Especially, one of the miRNAs was selected since it potently blocked autophagy induction by starvation. This miRNA was called hsa-miR-376b (or shortly miR-376b) of the miR-376 miRNA family. Subsequent tests in MCF-7 breast cancer cells confirmed initial screen results pointing out to an autophagy inhibitory effect of this miRNA (Fig. 1-A and Fig. 1-B).

For the discovery of potential miR-376 targets related to autophagy regulation, a combination of publicly available bioinformatic tools were used to predict miRNA binding sites using different approaches were chosen.

The analysis of miRNA predicted targets was determined by using the algorithms TargetScan (Lewis *et al,* 2005) (http://genes.mit.edu/targetscan/), microT (Maragkakis M *et al.,* 2009), MirGator (http://genome.ewha.ac.kr/miRGator/ miRGator.html), miRGen (Megraw M *et al.,* 2006), PITA (Probability of Interaction by Target Accessibility) (Kertesz *et al.,* 2007), and miRanda (http://cbio.mskcc.org/cgi-bin/mirnaviewer/mirnaviewer.pl) that each has a distinct pattern to search the interaction between the miRNA and target, such as evolutionary conservation, energy, or accessibility etc.

Bioinformatic analysis revealed Beclin1 and Atg4C genes as possible miR-376b targets. To confirm that both Beclin1 and Atg4C are indeed miR-376b targets, qPCR tests and immunobloting analyses of their expression were performed. MCF7 breast carcinoma cells were tranfected with plasmids coding for miR-376b or control hTR and grown in full (DMEM + 10%FCS) or starvation (Earl's Balance Salt Solution, EBSS) media. Beclin1 and Atg4C mRNA levels were analysed by qPCR tests. As shown in the Fig. 2-B (Beclin1 and Atg4C), overexpression of miR-376b, but not the control miRNA, downregulated the mRNA levels of both autophagy proteins in MCF-7 cells grown in full or starvation media. To check whether protein levels were affected by miRNA expression, immunoblots were performed using specific antibodies against Beclin1 or Atg4C (Fig. 2-C and Fig. 2-D). These immunoblot analyses demonstrated that protein levels of both autophagy proteins were also affected by miR-376b overexpression.

To prove that miRNA effects were not cell type specific, we performed similar qPCR and immunoblot analyses in the Huh-7 hepatocellular carcinoma cell line. Similar to the results in MCF-7 cells, mRNA levels of Beclin1 and Atg4c (Fig. 3-A) were significantly decreased in miR-376b transfected Huh-7 cells. Beclin1 and Atg4c (Fig. 3-B and Fig. 3-C) protein amounts were attenuated in mir-376b transfected cells but not in cells transfected with the control plasmid.

All these results indicated that miR-376b blocked autophagy by modulating the expression of two key proteins in the autophagy pathways, Beclin1 and Atg4c. miR-376b did so by affecting mRNA levels of both proteins. The decrease in Beclin1 and Atg4c protein levels might be the result of the effect on mRNA levels, yet, we cannot exclude a possible additive effect of this miRNA on protein translation.

A stable interaction due to partial base pairing between the miRNA seed region and its counterpart in the 3'UTR region, the seed match sequence, is crucial for miRNA action. Several destabilizing mutations were introduced to the seed match sequence in the putative miRNA binding site of BECLIN1 or ATGC 3'UTR and cloned these mutated miR-376 binding sites or the wild-type sequences to the 3'-UTR region of the luciferase cDNA in the pGL3 mammalian expression vector (Fig. 4-A and Fig. 4-B respectively).

Upon transfection to cells, luciferase activity jumped several logs above the backgorund level. Upon co-expression with the luciferase construct containing wild-type BECLIN1 (Fig. 4-C) or ATG4C (Fig. 4-D3'-UTR, miR-376b was able to attenuate dramatically the luciferase expression (measured by normalized luciferase activity).

In sharp contrast, luciferase constructs fused to mutant 3'-UTR of BECLIN1 or ATG4C were refractory to the effect of miR-376b. These results showed that the effect of miR-376b on the expression of autophagy proteins was direct and required the miRNA-binding sequences that were discovered in the 3'-UTR regions of BECLIN1 and ATG4C.

To test whether silencing endogenous miRNAs would have an impact on Beclin1 and Atg4c levels, antagomirs were designed against miR-376b. Cells were transfected with antagomir.

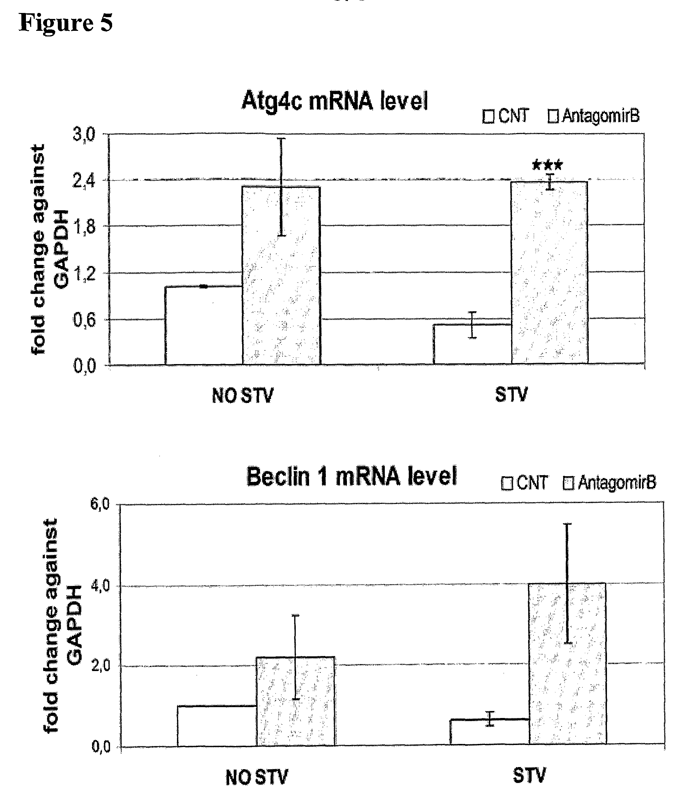

miR-376b or control antagomir, incubated cells in full or starvation media and, checked Beclin1 or Atg4c (Fig. 5) mRNA levels using qPCR. Transfection of antagomir-376b led to an increase in mRNAs of both autophagy proteins and the effect was more prominent following starvation. These results indicated that endogenous miR-376b controlled the levels of cellular Beclin1 and Atg4c.

Indeed, Beclin1 is a key autophagy protein and reports by several independent groups showed that Beclin 1 knockout or knockdown strongly blocked autophagy activated by various stimuli. As stated above, ubiquitously expressed protein Beclin1 binds to hVps34/class III PI3K and regulates the autophagy in mammalian cells (Liang X H *et al.,* 1999; and Furuya N *et al.,* 2005). Moreover, Beclin1 was shown to bind Bcl-2 family members (e.g. Bcl-2, Bcl-XL) and a balance between the levels of Bcl-2 proteins and Beclin was shown to affect autophagy activation (Pattingre S *et al.,* 2005). Downregulation of this protein or a change in the ratio of Bcl-2-free Beclin disturbs the initial stages of autophagosome formation (Hamasaki M, Yoshimori T 2010).

On the other hand, Atg4c/autophagin 3 belongs to family of cysteine proteases (Atg4a, b, c) that plays a role in the processing and lipidation of Atg8/LC3 and other Atg8-like proteins (i.e. GATE-16, GABARAP). Atg4 proteins are ubiquitously expressed in human tissues (Marino G. *et al* 2003). Atg4 proteins are also important regulators of autophagy and autophagic vesicle flow (Shouval R *et al.,* 2007). In line with this, knock-out of Atg4c was associated with autophagy inhibition (Marino G *et al.,* 2007).

Observations presented above demonstrated the potency of miR-376b in limiting autophagic activity. miR-376b does so by leading to a decrease in Beclin1 and Atg4c mRNA and protein levels. Indeed, miR376b affected Beclin1 and Atg4c levels through its direct action on their mRNAs, because the 3'UTR of these genes contained functional and specific miR376b binding sites and their mutation decreased the amplitude of the miRNA effect. Furthermore, inhibitors (antagomirs) of said miRNAs were able to suppress related miRNAs effect and increased the level of its targets. Therefore, usage of the miR-376b (or its derivatives) or anti miRNAs (antagomirs etc.) has the potential to provide possibilities to modulate and manipulate autophagy for medical purposes, hence to provide new diagnosis, treatment and follow-up approaches, and/or improve current protocols used against previously mentioned diseases with autophagy abnormalities.

### REFERENCES:

1. Kim J, Huang WP, Stromhaug PE, Klionsky DJ. J Biol Chem 2002; 277:763-73.
2. Gozuacik D, Kimchi A. Oncogene 2004; 23:2891-906.
3. Shintani T, Klionsky DJ. Science 2004; 306:990-5.
4. Ohsumi Y. Nat Rev Mol Cell Biol 2001; 2:211-6.
5. Mizushima N, Ohsumi Y, Yoshimori T. Cell Struct Funct 2002; 27:421-9.
6. Mizushima N, Levine B, Cuervo AM, Klionsky DJ. Nature 2008; 451, 1069-1075
7. Cuervo A.M., Trends in Cell Biology 2004;14:70-77
8. Mizushima N, Noda T, Yoshimori T, Tanaka Y, Ishii T, George MD, Klionsky DJ, Ohsumi M and Ohsumi Y. Nature 1998; 395:395-398.
9. Shintani T, Mizushima N, Ogawa Y, Matsuura A, Noda T and Ohsumi Y. Embo J. 1999; 18:5234-5241.
10. Tanida I, Mizushima N, Kiyooka M, Ohsumi M, Ueno T, Ohsumi Y and Kominami E.Mol. Biol. Cell. 1999; 10:1367-1379.
11. Mizushima N, Noda T and Ohsumi Y. Embo J. 1999;18:3888-3896.
12. Mizushima N, Kuma A, Kobayashi Y, Yamamoto A, Matsubae M, Takao T, Natsume T, Ohsumi Y and Yoshimori T. J. Cell Sci. 2003a; 116:1679-1688.
13. Ichimura Y, Kirisako T, Takao T, Satomi Y, Shimonishi Y, Ishihara N, Mizushima N, Tanida I, Kominami E, Ohsumi M, Noda T and Ohsumi Y. Nature 2000;408:488-492.
14. Kirisako T, Baba M, Ishihara N, Miyazawa K, Ohsumi M, Yoshimori T, Noda T and Ohsumi Y. J. Cell Biol. 1999;147:435-446.
15. Kirisako T, Ichimura Y, Okada H, Kabeya Y, Mizushima N, Yoshimori T, Ohsumi M, Takao T, Noda T and Ohsumi Y. J. Cell Biol. 2000; 151:263-276.
16. Reggiori F, Tucker KA, Stromhaug PE, Klionsky DJ, Developmental cell 2004; 6:79-90
17. Rami A, Langhagen A, Steiger S. Neurobiology of Disease 2008;29:132-141
18. Darsow T., Rieder S.E., Emr S.D. J Cell Biol. 1997;138:517-529.
19. Kim I., Rodriguez-Enriquez S., Lemasters J.J. Arch Biochem Biophys 2007;462:245-253.
20. Aita VM, Liang HX, Murty VVVS, Pincus DL, Yu W, Cayanis E, Kalachikov S, Gilliam TC, Levine B. Genomics 1999;59(1):59-65.
21. Liang XH, Jackson S, Seaman M, Brown K, Kempkes B, Hibshoosh H, Levine B. Nature 1999;402:672-6.
22. Wang ZH, Peng ZL, Duan ZL, Liu H. Sichuan Da Xue Xue Bao Yi Xue Ban. 2006;37:860-3.
23. Wang ZH, Peng ZL, Duan ZL, Liu H. Sichuan Da Xue Xue Bao Yi Xue Ban. 2007; 38: 239-42.
24. Miracco C, Cosci E, Oliveri G, Luzi P, Pacenti L, Monciatti I, Mannucci S, De Nisi MC, Toscano M, Malagnino V, Falzarano SM, Pirtoli L, Tosi P. Int. J. Oncol. 2007;30:429-36.
25. Koneri K, Goi T, Hirono Y, Katayama K, Yamaguchi A. Anticancer Res. 2007;27:1453-7.
26. Ambros V. Nature 2004;431(7006):350-5.
27. Lewis B.P., Burge C.B. and Bartel D.P. Cell 2005;120:15-20.
28. Zhu H et al. Autophagy 2009;5(6):816-23.
29. Kircher M, Bock C and Paulsen M. BMC Genomics 2008; 9:346
30. Hamasaki M, Yoshimori T. FEBS Letters 2010; 584:1296-1301
31. ImageJ, website: http://rsb.info.nih.gov/ij/, Research Services Branch of The National Institute of Health, USA.
32. miRanda, website: http://cbio.mskcc.ora/cai-bin/mirnaviewer/mirnaviewer.pl, Computational Biology Center of Memorial Sloan-Kettering Cancer Center.
33. MirGator, website: http://genome.ewha.ac.kr/miRGator/miRGator.html, Lab. of Bioinformatics, Ewha Womans University 11-1 Daehyun-dong, Seodaemun-gu, Seoul, 120-750, Korea.
34. Seitz H, Royo H, Bortolin ML, Lin SP, Anne C. Ferguson-Smith, and Jérôme Cavaillé. Genome Res. 14, 1741-1748 (2004).
35. TargetScan, website: http://genes.mit.edu/targetscan/, Whitehead Institute for Biomedical Research-MIT.
36. Mariño G et al. J Biol Chem 2003;278(6):3671-8
37. Mariño G et al. J. Biol. Chem. 2007; 282:18573-18583
38. Maragkakis M.; Reczko M.; Simossis V. A.; Alexiou P.; Papadopoulos G. L.; Dalamagas T.; Giannopoulos G.; Goumas G.; Koukis E.; Kourtis K.; Vergoulis T.; Koziris N.; Sellis T.; Tsanakas P.; Hatzigeorgiou A. G. Nucleic Acids Research 2009.
39. Vellai T, Cell Death and Differentiation 2009;16:94-102
40. Furuya N, Yu J, Byfield M, Pattingre S, Levine B. Autophagy 2005; 1(1):46-52
41. R J Palomino-Morales, J Oliver, M Gómez-García, M A López-Nevot, L Rodrigo, A Nieto, B Z Alizadeh and J Martín, Genes and Immunity 2009; 10, 356-364
42. Pattingre S, Tassa A, Qu X, Garuti R, Liang XH, Mizushima N, Packer M, Schneider MD and Levine B, Cell 2005; 22(6):927-939.
43. Shouval RS, Shvets E, Fass E, Shorer H, Gil L, Elazar Z. EMBO J. 2007;26(7):1749-1760.
44. Melnikova I, Nature Reviews in Drug Discovery 2007; 6(5):341-2.
45. Megraw M., Sethupathy P., Corda B., and Hatzigeorgiou A.G. (2006). miRGen: A database for the study of animal microRNA genomic organization and function. Nucleic Acids Research, 35: D149-D155.
46. Kertesz M, lovino N, Unnerstall U, Gaul U and Segal E (2007) The role of site accessibility in microRNA target recognition. Nat Genet 39:1278-84.
47. Cadwell K, Liu JY, Brown SL, Miyoshi H, Loh J, Lennerz JK, Kishi C, Kc W, Carrero JA, Hunt S, Stone CD, Brunt EM, Xavier RJ, Sleckman BP, Li E, Mizushima N, Stappenbeck TS, Virgin HW 4th. Nature. 2008; 456(7219):259-63
48. Poy MN, Eliasson L, Krutzfeldt J, Kuwajima S, Ma X, Macdonald PE, Pfeffer S, Tuschl T, Rajewsky N, Rorsman P, Stoffel M. Nature. 2004;432(7014):226-30.

### SEQUENCES

| ID 1 | Accesion | Mature sequence |
|---|---|---|
| hsa-miR-376b | MIMAT0002172 | AUCAUAGAGGAAAAUCCAUGUU |
| | | |

| ID 2 | Accession | Stem-loop Sequence |
|---|---|---|
| hsa-miR-376b | MI0002466 | CAGUCCUUCUUUGGUAUUUAAAACGUGGAU AUUCCUUCUAUGUUUACGUGAUUCCUGGUU AAUCAUAGAGGAAAAUCCAUGUUUCAGUA UCAAAUGCUG |

## Claims

1. A miR-376b (hsa-miR-376b) or an inhibitor thereof, for use in treatment of at least one disease involving autophagy abnormalities alone or in combination with other conventional treatments of diseases, where said inhibitor is a nucleic acid molecule comprising a sequence that is at least 70% complementary to a contiguous 5' to 3' sequence of a mature miRNA,.

2. Use of miR-376b (hsa-miR-376b) for diagnosis of at least one disease involving autophagy abnormalities.

3. The miR-376b for use according to claim 1, **characterized in that**; said miR-376b is used in blocking autophagy in case the disease arises with excessive autophagy.

4. The miR-376b for use according to claim 3, **characterized in that**; said miR-376b is used to suppress at least one autophagy related gene for inhibition of the autophagy.

5. The miR-376b inhibitor for use according to claim 1, **characterized in that**; said miR-376b inhibitor is used to activate autophagy in case the disease arises from the absence or deficiency of autophagy.

6. The miR-376b inhibitor for use according to claim 1 or 5, **characterized in that**; said miR-376b inhibitor is used to suppress at least one miRNA of miRNA-376 family for initiation of the autophagy.

7. The miR-376b for use according to claim 4 wherein the autophagy related gene is at least one of Beclin 1 and Atg4c.

8. The miR-376b for use according to claim 1, wherein miR-376b is a naked synthetic RNA.

9. The miR-376b for use according to claim 1, wherein miR-376b is a chemically modified synthetic RNA.

10. The miR-376b for use according to claim 9, wherein the synthetic RNA is modified with a chemical moiety selected from the group consisting of phosphorothioate, boranophosphate, 2'-O-methyl, 2'-fluoro, , 2'-O-methoxyethyl (2'-O-MOE), 2'-O-aminopropyl (2'-O-AP), 2'-O-dimethylaminoethyl (2'-O-DMAOE), 2'-O-dimethylaminopropyl (2'-O-DMAP), 2'-O-dimethylaminoethyloxyethyl (2'-O-DMAEOE), and 2'-O-N-methylacetamido (2'-O-NMA), PEG, terminal inverted-dT base, fluoro-β-d-arabinonucleic acid (FANA or as 4'-S-FANA) or arabinonucleic acid (ANA) modifications, the addition of lauric acid, lithocholic acids and cholesterol derivatives and combinations thereof.

11. The miR-376b for use according to claim 1 or 2, wherein the miR-376b is comprised in a liposome, polymer-based nanoparticle, cholesterol conjugate, cyclodextran complex, polyethylenimine polymer or a protein complex.

12. The miR-376b for use according to claim 1, wherein the miR-376b is in a dosage form to be administered directly to the diseased tissue in the organism, intravenously, subcutaneously, intramuscularly, nasally, intraperitonealy, vaginally, anally, orally, intraocularly or intrathecally.

13. The miR-376b inhibitor for use according to claim 1 wherein the miR-376b inhibitor is between 12 and 30 nucleotides in length.

14. The miR-376b inhibitor for use according to claim 1 wherein the miR-376b inhibitor comprises a modification selected from the group consisting of: 2'-deoxy, 2'-deoxy-2'-fluoro, 2'-O-methyl, 2'-O-methoxyethyl (2'-O-MOE), 2'-O-aminopropyl (2'-O-AP), 2'-O-dimethylaminoethyl (2'-O-DMAOE), 2'-O-dimethylaminopropyl (2'-O-DMAP), 2'-O-dimethylaminoethyloxyethyl (2'-O-DMAEOE), and 2'-O-N-methylacetamido (2'-O-NMA), PEG, terminal inverted-dT base, fluoro-β-d-arabinonucleic acid (FANA or as 4'-S-FANA) or arabinonucleic acid (ANA) modifications, the addition of lauric acid, lithocholic acids and cholesterol derivatives and combinations.

15. The miR-376b for use according to claim 1 or 2 wherein the disease is selected from a group consisting of neurodegeneration, cancer, hearth diseases, liver diseases, ageing, myopathies, auto-immune, inflammatory diseases, infectious diseases, ischemic diseases, immune deficiencies, diabetes, axonal injury, lysosomal storage diseases and nervous system diseases.

16. The miR-376b for use according to claim 1 or 2 wherein the disease is breast or liver cancer.

## Patentansprüche

1. miR-376b (hsa-miR-376b) oder ein Inhibitor davon, zur Verwendung bei der Behandlung von mindestens einer Krankheit, die Autophagie-Anomalien beinhaltet, allein oder in Kombination mit anderen herkömmlichen Behandlungen von Krankheiten, wobei der Inhibitor ein Nukleinsäuremolekül ist, das eine Sequenz umfasst, die zu einer durchgehenden 5'-nach-3'-Sequenz einer reifen miRNA mindestens 70% komplementär ist.

2. Verwendung von miR-376b (hsa-miR-376b) zur Diagnose von mindestens einer Krankheit, die Autophagie-Anomalien beinhaltet.

3. miR-376b zur Verwendung nach Anspruch 1, **dadurch gekennzeichnet, dass** miR-376b zur Blockierung von Autophagie verwendet wird, wenn die Krankheit mit übermäßiger Autophagie auftritt.

4. miR-376b zur Verwendung nach Anspruch 3, **dadurch gekennzeichnet, dass** miR-376b zur Unterdrückung von mindestens einem mit Autophagie in Zusammenhang stehenden Gen zur Hemmung der Autophagie verwendet wird.

5. miR-376b-Inhibitor zur Verwendung nach Anspruch 1, **dadurch gekennzeichnet, dass** der miR-376b-Inhibitor zur Aktivierung von Autophagie verwendet wird, wenn sich die Krankheit aufgrund des Fehlens oder der Defizienz von Autophagie ergibt.

6. miR-376b-Inhibitor zur Verwendung nach Anspruch 1 oder 5, **dadurch gekennzeichnet, dass** der miR-376b-Inhibitor zur Unterdrückung von mindestens einer miRNA der miRNA-376-Familie zur Initialisierung von Autophagie verwendet wird.

7. miR-376b zur Verwendung nach Anspruch 4, wobei das mit Autophagie in Zusammenhang stehende Gen mindestens eines von Beclin 1 und Atg4c ist.

8. miR-376b zur Verwendung nach Anspruch 1, wobei miR-376b eine nackte synthetische RNA ist.

9. miR-376b zur Verwendung nach Anspruch 1, wobei miR-376b eine chemisch modifizierte synthetische RNA.

10. miR-376b zur Verwendung nach Anspruch 9, wobei die synthetische RNA mit einer chemischen Einheit modifiziert ist, ausgewählt aus der Gruppe, bestehend aus Phosphorthioat-, Boranophosphat-, 2'-O-Methyl-, 2'-Fluor-, 2'O-Methoxyethyl- (2'-O-MOE), 2'-O-Aminopropyl- (2'-O-AP), 2'-O-Dimethylaminoethyl- (2'-O-DMAOE), 2'-O-Dimethylaminopropyl- (2'-O-DMAP), 2'-O-Dimethylaminoethyloxyethyl- (2'-O-DMAEOE) und 2'-O-N-Methylacetamido- (2'-O-NMA), PEG-, terminaler invertierter-dT-Base-, Fluor-β-d-arabinonukleinsäure- (FANA oder als 4'-S-FANA) oder Arabinonukleinsäure- (ANA) - Modifikationen, der Zugabe von Laurinsäure, Lithocholsäuren und Cholesterinderivaten und Kombinationen davon.

11. miR-376b zur Verwendung nach Anspruch 1 oder 2, wobei miR-376b in einem Liposom, Nanopartikel auf Polymerbasis, Cholesterinkonjugat, Cyclodextrankomplex, Polyethyleniminpolymer oder einem Proteinkomplex enthalten ist.

12. miR-376b zur Verwendung nach Anspruch 1, wobei miR-376b in einer Dosierungsform vorliegt, die intravenös, subcutan, intramuskulär, nasal, intraperitoneal, vaginal, anal, oral, intraokular, oder intrathecal direkt an das erkrankte Gewebe in dem Organismus verabreicht werden soll.

13. miR-376b-Inhibitor zur Verwendung nach Anspruch 1, wobei der miR-376b-Inhibitor zwischen 12 und 30 Nukleotide lang ist.

14. miR-376b-Inhibitor zur Verwendung nach Anspruch 1, wobei der miR-376b-Inhibitor eine Modifikation umfasst, ausgewählt aus der Gruppe, bestehend aus 2'-Desoxy-, 2'-Desoxy-2'-fluor-, 2'-O-Methyl-, 2'O-Methoxyethyl- (2'-O-MOE), 2'-O-Aminopropyl- (2'-O-AP), 2'-O-Dimethylaminoethyl- (2'-O-DMAOE), 2'-O-Dimethylaminopropyl- (2'-O-DMAP), 2'-O-Dimethylaminoethyloxyethyl- (2'-O-DMAEOE) und 2'-O-N-Methylacetamido- (2'-O-NMA), PEG-, terminaler invertierter-dT-Base, Fluor-β-d-arabinonukleinsäure- (FANA oder als 4'-S-FANA) oder Arabinonukleinsäure- (ANA) -Modifikationen, der Zugabe von Laurinsäure, Lithocholsäuren und Cholesterinderivaten und Kombinationen davon.

15. miR-376b zur Verwendung nach Anspruch 1 oder 2, wobei die Krankheit ausgewählt ist aus einer Gruppe, bestehend aus Neurodegeneration, Krebs, Herzkrankheiten, Leberkrankheiten, Alterung, Myopathien, Autoimmun-, Entzündungskrankheiten, Infektionskrankheiten, ischämischen Krankheiten, Immundefizienzen, Diabetes, Axonverletzung, lysosomalen Speicherkrankheiten, und Krankheiten des Nervensystems.

16. miR-376b zur Verwendung nach Anspruch 1 oder 2, wobei die Krankheit Brust- oder Leberkrebs ist.

## Revendications

1. Un miR-376b (hsa-miR-376b) ou un inhibiteur de ce dernier, pour une utilisation dans le traitement d'au moins une maladie impliquant des anomalies de l'autophagie seul ou en combinaison avec d'autres traitements conventionnels de maladies, dans lequel ledit inhibiteur est une molécule d'acide nucléique comprenant une séquence qui est complémentaire à au moins 70 % d'une séquence 5' à 3' contiguë d'un miRNA mature.

2. Utilisation de miR-376b (hsa-miR-376b) pour diagnostiquer au moins une maladie impliquant des anomalies de l'autophagie.

3. Le miR-376b pour une utilisation selon la revendication 1, **caractérisé en ce que** ledit miR-376b est utilisé dans le blocage de l'autophagie dans le cas où la maladie résulte d'une autophagie excessive.

4. Le miR-376b pour une utilisation selon la revendication 3, **caractérisé en ce que** ledit miR-376b est utilisé afin de supprimer au moins un gène associé à l'autophagie pour inhiber l'autophagie.

5. L'inhibiteur de miR-376b pour une utilisation selon la revendication 1, **caractérisé en ce que** ledit inhibiteur de miR-376b est utilisé pour activer l'autophagie dans le cas où la maladie résulte de l'absence ou de la déficience d'autophagie.

6. L'inhibiteur de miR-376b pour une utilisation selon la revendication 1 ou 5, **caractérisé en ce que** ledit inhibiteur de miR-376b est utilisé afin de supprimer au moins un miRNA de la famille miRNA-376 pour initier l'autophagie.

7. Le miR-376b pour une utilisation selon la revendication 4, dans lequel le gène associé à l'autophagie est au moins l'un parmi Beclin 1 et Atg4c.

8. Le miR-376b pour une utilisation selon la revendication 1, dans lequel miR-376b est un ARN nu synthétique.

9. Le miR-376b pour une utilisation selon la revendication 1, dans lequel miR-376b est un ARN synthétique chimiquement modifié.

10. Le miR-376b pour une utilisation selon la revendication 9, dans lequel l'ARN synthétique est modifié avec une fraction chimique sélectionnée parmi le groupe constitué de phosphorothioate, boranophosphate, 2'-O-méthyl, 2'-fluoro, 2'-O-méthoxyéthyl (2'-O-MOE), 2'-O-aminopropyl (2'-O-AP), 2'-O-diméthylaminoéthyl (2'-O-DMAOE), 2'-O-diméthylaminopropyl (2'-O-DMAP), 2'-O-diméthylaminoéthyloxyéthyl (2'-O-DMAEOE), et 2'-O-N-méthylacétamido (2'-O-NMA), PEG, base dT terminale inversée, modifications de l'acide fluoro-β-d-arabinonucléique (FANA ou en tant que 4'-S-FANA) ou d'acide arabinonucléique (ANA), ajout d'acide laurique, d'acides lithocholiques et de dérivés de cholestérol et les combinaisons de ceux-ci.

11. Le miR-376b pour une utilisation selon la revendication 1 ou 2, dans lequel le miR-376b est compris dans un liposome, une nanoparticule à base de polymère, un conjugué de cholestérol, un complexe de cyclodextrane, un polymère polyéthylèneimine ou un complexe de protéines.

12. Le miR-376b pour une utilisation selon la revendication 1, dans laquelle le miR-376b est sous une forme posologique destinée à être administrée directement au tissu malade dans l'organisme, par voie intraveineuse, sous-cutanée, intramusculaire, nasale, intrapéritonéale, vaginale, anale, orale, intraoculaire ou intrathécale.

13. L'inhibiteur de miR-376b pour une utilisation selon la revendication 1, dans lequel l'inhibiteur de miR-376b a une longueur comprise entre 12 et 30 nucléotides.

14. L'inhibiteur de miR-376b pour une utilisation selon la revendication 1, dans lequel l'inhibiteur de miR-376b comprend une modification sélectionnée parmi le groupe constitué de : 2'-deoxy, 2'-deoxy-2'-fluoro, 2'-O-méthyle, 2'-O-méthoxyéthyle (2'-O-MOE), 2'-O-aminopropyle (2'-O-AP), 2'-O-diméthylaminoéthyle (2'-O-DMAOE), 2'-O-diméthylaminopropyle (2'-O-DMAP), 2'-O-diméthylaminoéthyloxyéthyle (2'-O-DMAEOE), et 2'-O-N-méthylacétamido (2'-O-NMA), PEG, base dT terminale inversée, modifications d'acide fluoro-β-d-arabinonucléique (FANA ou en tant que 4'-S-FANA) ou d'acide arabinonucléique (ANA), ajout d'acide laurique, d'acides lithocholiques et de dérivés de cholestérol et de combinaisons de ceux-ci.

15. Le miR-376b pour une utilisation selon la revendication 1 ou 2, dans lequel la maladie est sélectionnée parmi un groupe constitué de neurodégénérescence, de cancer, de maladies cardiaques, de maladies hépatiques, de vieillissement, de myopathies, de maladies inflammatoires et auto-immunes, de maladies infectieuses, de maladies ischémiques, de déficiences immunitaires, de diabète, de lésion de l'axone, de maladies de stockage lysosomal et de maladies du système nerveux.

16. Le miR-376b pour une utilisation selon la revendication 1 ou 2, dans lequel la maladie est le cancer du sein ou du foie.
